# EUROPEAN PATENT APPLICATION

(11) **EP 4 834 354 A1**
(43) Date of publication of application: **04.11.2026**
(21) Application number: 23963064.3
(22) Date of filing: 26.12.2023
(51) Int. Cl.: A61K 35/742, A61P 37/02, A61P 37/04, A61P 37/06, A61P 43/00

(54) **NEUTROPHIL-ACTIVATION-REGULATING COMPOSITION CONTAINING SACCHARIFICATION BACTERIA**

(71) Applicant: Toa Biopharma Co., Ltd., Tokyo 151-0073 (JP)
(72) Inventor: Hayashi Takuto, Tokyo 151-0073 (JP); Nakagomi Rena, Tokyo 151-0073 (JP); Okino Yoichi, Tokyo 151-0073 (JP)
(74) Representative: Krauss, Jan
(86) International application number: PCT/JP2023/046713
(87) International publication number: WO 2025/141710

(57) **Abstract**

A neutrophil activation-regulating composition having both functions of promoting and/or suppressing neutrophil extracellular trap (NET) formation is provided. It has been confirmed that amylolytic bacteria (particularly Bacillus subtilis) can promote and/or suppress NET formation in neutrophil-like cells.

## Description

### TECHNICAL FIELD

The present disclosure relates to a neutrophil activation-regulating composition containing amylolytic bacteria.

### BACKGROUND ART

There are many types of immune cells, but neutrophils make up more than half of the immune cells in human peripheral blood. Neutrophils defend the body against foreign microorganisms by various means. One such means is neutrophil extracellular traps (NETs) (Non-Patent Literature 1). NETs are a phenomenon in which neutrophils release a web-like structure composed of DNA and proteins to the outside of the cell to capture the microorganisms during microbial infection. This is an innate immune function that prevents local infection from spreading throughout the body. In NET formation, the swelling of chromatin and the rupture of the cell membrane following the disruption of the neutrophil nuclear membrane are characteristic, and this series of cell death processes is called NETosis.

On the other hand, it has recently been reported that NETosis also occurs in various inflammatory diseases other than microbial infection. For example, in respiratory diseases such as acute lung injury (ALI), acute respiratory distress syndrome (ARDS), chronic obstructive pulmonary disease (COPD), and cystic fibrosis (CF); thrombotic diseases such as antiphospholipid syndrome (APS), stroke, and thrombotic microangiopathy (TMA); autoimmune diseases such as systemic lupus erythematosus (SLE), anti-neutrophil cytoplasmic antibody-associated vasculitis (AAV), and rheumatoid arthritis (RA); cancer; and sepsis, it has been suggested that NET formation may induce excessive inflammation (Non-Patent Literature 2).

Furthermore, when histones, myeloperoxidase, and elastase, which are constituent components of NETs, are released into the host's blood and tissues, they also become damaging factors for the host tissues and cells.

As described above, NET formation or the occurrence of NETosis is an innate immune function with a dual nature of host defense and inflammation exacerbation, and it is desirable that it be appropriately controlled.

To date, several substances that regulate neutrophil activation have been reported.

For example, a neutrophil activation regulator containing a thrombin-like enzyme as an active ingredient has been reported, which inhibits neutrophil degranulation, Mac-1 expression, NET formation (Patent Literature 1).

In addition, histidine-rich glycoprotein, which is synthesized in the liver and present in plasma and is known to be involved in regulating the coagulation and fibrinolysis system and controlling angiogenesis, has been reported to be neutrophil activation regulator (Patent Literature 2).

Furthermore, it has been reported that many pathogenic microorganisms induce NETs thereby activate neutrophils (Non-Patent Literature 3). In addition, Lacticaseibacillus rhamnosus GG strain, a type of probiotic lactic acid bacterium, is known to suppress NET formation (Non-Patent Literature 4). It is also known that neutrophil-like HL-60 cells differentiated from human leukemia cell line HL-60 are often used as a model when evaluating the effect on NET formation in vitro (Non-Patent Literature 5).

### CITATION LIST

### Patent Literature

[Patent Literature 1] Japanese Patent No. 7100854
[Patent Literature 2] Japanese Patent No. 5807937

### Non-Patent Literature

[Non-Patent Literature 1] Brinkmann V et al: Neutrophil extracellular traps kill bacteria. Science. 303:1532-1535, 2004
[Non-Patent Literature 2] Sollberger G et al: Neutrophil extracellular traps: The biology of chromatin externalization. Dev.Cell. 44(5):542-553, 2018
[Non-Patent Literature 3] Mitsios A et al: NETopathies? Unraveling the dark side of old diseases through neutrophils. Front. Immunol. 7(678):678, 2017
[Non-Patent Literature 4] Vong L et al: Probiotic Lactobacillus rhamnosus inhibits the formation of neutrophil extracellular traps. J. Immunol. 192(4):1870-1877, 2014
[Non-Patent Literature 5] Manda-Handzlik A et al: The influence of agents differentiating HL-60 cells toward granulocyte-like cells on their ability to release neutrophil extracellular traps. Immunol. Cell Biol. 96(4):413-425, 2018

### SUMMARY OF INVENTION

### Technical Problem

As described above, NET formation or the occurrence of NETosis is an innate immune function with dual nature of host defense and inflammation exacerbation, and it is desirable that it be appropriately controlled. However, there are no reports of a substance that can control NETs in bidirectional manner, either promoting or suppressing it.

Therefore, an object of the present disclosure is to provide a neutrophil activation-regulating composition that has a bidirectional function of promoting and/or suppressing of NET formation.

The inventors have conducted intensive research to solve the above problem, and as a result, confirmed that amylolytic bacteria (particularly Bacillus subtilis) can promote and/or suppress NET formation in neutrophil-like cells, thereby completing the present disclosure.

The present disclosure is as follows:
1. A neutrophil activation-regulating composition containing amylolytic bacteria.
2. The composition according to item 1, wherein the amylolytic bacteria belong to the genus Bacillus.
3. The composition according to item 1, wherein the amylolytic bacteria are of the Bacillus subtilis species.
4. The composition according to item 1, wherein the amylolytic bacterium is Bacillus subtilis TO-A.
5. The composition according to item 3 or 4, wherein the neutrophil activation-regulating is promotion and/or suppression of neutrophil NET formation.
6. The composition according to item 1, wherein the activation-regulating is promotion.
7. A therapeutic agent for a disease caused by insufficient neutrophil activation, containing the composition according to item 6.
8. The composition according to item 1, wherein the activation-regulating is suppression.
9. A therapeutic agent for a disease caused by excessive neutrophil activation, containing the composition according to item 8.
10. A method for regulating neutrophil activity, containing the step of administering the composition according to any one of items 1 to 4 to mammals other than humans.
11. A method for treating a disease caused by excessive neutrophil activation, containing the step of administering amylolytic bacteria to a target subject.
12. A method for treating a disease caused by insufficient neutrophil activation, containing the step of administering amylolytic bacteria to a target subject.
13. Use of amylolytic bacteria for the manufacture of a neutrophil activation-regulating composition.
14. Use of a neutrophil activation-regulating composition containing amylolytic bacteria.

### Advantageous Effects of Invention

The composition of the present disclosure can promote and/or suppress neutrophil NET formation.

### BRIEF DESCRIPTION OF THE DRAWINGS

[FIG. 1A] FIG. 1A shows neutrophil-like HL-60 cells.
[FIG. 1B] FIG. 1Bshows the result of inducing NET release by treating neutrophil-like HL-60 cells with phorbol 12-myristate 13-acetate (PMA), a NET inducer, for 3 hours.
[FIG. 1C] FIG. 1C shows the result obtained after degrading DNA with DNaseI following treatment of neutrophil-like HL-60 cells with PMA for 3 hours.
[FIG. 2] FIG. 2 shows the measurement results of the amount of NET release after treating neutrophil-like HL-60 cells, which had been exposed to Bacillus subtilis TO-A strain, with PMA for 3 hours.

### DESCRIPTION OF EMBODIMENTS

### (Subject of the Present Disclosure)

The present disclosure relates to a neutrophil activation-regulating composition, a therapeutic agent for a disease caused by insufficient neutrophil activation, a therapeutic agent for a disease caused by excessive neutrophil activation, and a method for regulating neutrophil activity.

The present disclosure will be described in detail below. The composition of the present disclosure is not particularly limited, and can be formulated by a well-known method into powders, tablets, granules, capsules, liquid formulations, and the like, by blending additives such as known carriers (e.g., starch, lactose, and soy protein), as well as excipients, binders, disintegrants, lubricants, stabilizers, and suspending agents.

Furthermore, the present disclosure relates to a prescription drug, an over-the-counter drug, a quasi-drug, a veterinary drug, a feed, a feed additive, a food for specified health uses, a food with nutrient function claims, a food with function claims, a nutritional drink, or the like, which contain the above composition.

In addition, in the present specification, when preferable numerical ranges (for example, ranges of content or weight-average molecular weight) are described in a stepwise manner, the respective lower limit values and upper limit values can be combined independently of one another. For example, in the description "preferably from 10 to 100, more preferably from 20 to 90," the "preferable lower limit value: 10" and the "more preferable upper limit value: 90" can be combined to give "from 10 to 90."

### (Neutrophil Activation)

The "neutrophil activation" in the present disclosure refers to an action (phenomenon) exerted by neutrophils upon stimulation by neutrophil activating factors, and examples thereof include NET formation, degranulation, production of reactive oxygen species, Mac-1 expression, transendothelial migration, and tissue infiltration.

The "neutrophil activation-regulating" in the present disclosure particularly means promotion and/or suppression of NET formation, but does not imply exclusion of the other phenomena described above.

"NET formation" means the formation of a web-like structure composed of DNA and proteins by the swelling of chromatin and the rupture of the cell membrane following the disruption of the neutrophil nuclear membrane.

### (Therapeutic Agent for a Disease Caused by Excessive Neutrophil Activation)

The "therapeutic agent for a disease caused by excessive neutrophil activation" in the present disclosure means a therapeutic agent can be used for diseases caused by tissues and organ damage resulting from neutrophils carrying out NET formation in excess of that required for host defense, or from the production of excessive reactive oxygen species, cytokines, antimicrobial peptides, or proteolytic enzymes.

Specific examples of such diseases include respiratory diseases such as acute lung injury, acute respiratory distress syndrome, chronic obstructive pulmonary disease, and cystic fibrosis; thrombotic diseases such as antiphospholipid syndrome, stroke, and thrombotic microangiopathy; autoimmune diseases such as systemic lupus erythematosus, anti-neutrophil cytoplasmic antibody-associated vasculitis, and rheumatoid arthritis; cancer; and sepsis.

The target subject for administration includes patients suffering from the above-described diseases and patients who require prevention of such diseases.

### (Therapeutic Agent for a Disease Caused by Insufficient Neutrophil Activation)

The "therapeutic agent for a disease caused by insufficient neutrophil activation" in the present disclosure means a therapeutic agent that can be used for diseases caused by insufficient NET formation by neutrophils, or insufficient production of reactive oxygen species, cytokines, antimicrobial peptides, or proteolytic enzymes when the body is infected with pathogenic microorganisms such as bacteria, viruses, fungi, mycoplasma, or parasites.

Specific examples of such diseases include infectious diseases such as bacterial infections caused by Streptococcus, Staphylococcus aureus, methicillin-resistant Staphylococcus aureus (MRSA), pathogenic Escherichia coli, and Pseudomonas aeruginosa; viral infections such as influenza, viral pneumonia, viral hepatitis, viral gastroenteritis, cytomegalovirus infection, and Japanese encephalitis; fungal infections such as aspergillosis and candidiasis; and parasitic infections such as schistosomiasis japonica, filariasis, and ascariasis.

The target subject for administration includes patients suffering from the above-described diseases and patients who require prevention of such diseases.

### (Composition of the Present Disclosure)

The composition of the present disclosure is a composition containing amylolytic bacteria as an essential component.

The amylolytic bacteria (Amylolytic Bacillus) are preferably of the genus Bacillus, more preferably Bacillus subtilis, Bacillus mesentericus, or Bacillus polyfermenticus, and even more preferably Bacillus subtilis TO-A.

Furthermore, bacteria of the genus Bacillus are spore-forming, obligately aerobic (partially facultatively anaerobic), Gram-positive bacilli. When spore-forming bacteria are placed under environments with poor conditions such as nutrients and temperatures, or come into contact with compounds toxic to the bacteria, spores are formed inside the bacterial cells. For this reason, spores are sometimes called the durable form or dormant form of the bacteria. When the spores are placed under environment suitable for proliferation of the bacteria, or under stress conditions such as heat, e.g., a heat shock, the spores germinate and become vegetative cells or proliferative cells having normal proliferative and metabolic capacities. When spore-form amylolytic bacteria enter the body, they germinate into different numbers of vegetative cells depending on the different environments, and then exert the function of regulating neutrophil activation.

The above-described bacteria may be live bacteria or dead bacteria but are preferably viable bacteria. The bacteria may also be processed as any treated material or extraction residue, for example, an ultrasonically disrupted material, a homogenizer-disrupted material, a methanol extraction residue, or an ethyl acetate extraction residue.

In addition, the composition of the present disclosure may be in the form of a powder, granules, an orally disintegrating tablet, a plain tablet, a coated tablet, a capsule, a suspension, or a liquid formulation.

The composition of the present disclosure is available from Toa Biopharma Co., Ltd. (reference: http://www.toabio.co.jp/medicalitem). In addition, Bacillus subtilis TO-A is available from the Patent Microorganisms Depositary of the National Institute of Technology and Evaluation as a bacterial strain having the unique accession number designated as FERM BP-07462.

Furthermore, in the composition of the present disclosure, in addition to the amylolytic bacteria, it can be formulated by blending known carriers (e.g., starch, lactose, and soy protein), pharmaceutically or alimentarily acceptable excipients, protective agents, buffering agents, tonicity agents, flavoring agents, colorants, binders, disintegrants, lubricants, stabilizers, suspending agents, coating agents, and the like.

### (Content of Amylolytic Bacteria in the Composition of the Present Disclosure)

Based on the results of Example 2 described below, the composition of the present disclosure can be made into a "neutrophil activation-promoting composition" by setting the content of amylolytic bacteria (particularly vegetative cells) in the composition to a low concentration, and can be made into a "neutrophil activation-suppressing composition" by setting the content to a high concentration.

That is, the composition of the present disclosure can promote neutrophil activation when the activation state of neutrophils is low (i.e., when there are few activated neutrophils), whereas it can suppress neutrophil activation when the activation state of neutrophils is too high (i.e., when there are too many activated neutrophils).

For example, the content of amylolytic bacteria in the composition of the present disclosure (particularly, the content in an aqueous solution, and further the content in a cell suspension) can be set as follows.

The bacterial content in the neutrophil activation-regulating composition is 1.0 × 10⁴ to 1.0 × 10¹¹ cells/mL, or 5.0 × 10⁴ to 1.0 × 10¹⁰ cells/mL, and more preferably 1.0 × 10⁵ to 6.0 × 10⁸ cells/mL.

The bacterial content in the neutrophil activation-promoting composition is 1.0 × 10⁴ to 2.0 × 10⁶ cells/mL, 1.0 × 10⁴ to 2.1 × 10⁶ cells/mL, 1.0 × 10⁶ to 2.2 × 10⁶ cells/mL, 1.0 × 10⁴ to 2.3 × 10⁶ cells/mL, or 1.0 × 10⁴ to 2.4 × 10⁶ cells/mL, and is preferably 5.0 × 10⁴ to 2.0 × 10⁶ cells/mL, 5.0 × 10⁴ to 2.1 × 10⁶ cells/mL, 5.0 × 10⁴ to 2.2 × 10⁶ cells/mL, 5.0 × 10⁴ to 2.3 × 10⁶ cells/mL, 5.0 × 10⁴ to 2.4 × 10⁶ cells/mL, 1.0 × 10⁴ to 1.5 × 10⁶ cells/mL, 1.0 × 10⁴ to 1.6 × 10⁶ cells/mL, 1.0 × 10⁴ to 1.7 × 10⁶ cells/mL, 1.0 × 10⁴ to 1.8 × 10⁶ cells/mL, or 1.0 × 10⁴ to 1.9 × 10⁶ cells/mL, 5.0 × 10⁴ to 1.4 × 10⁶ cells/mL, 5.0 × 10⁴ to 1.3 × 10⁶ cells/mL, 5.0 × 10⁴ to 1.2 × 10⁶ cells/mL, 5.0 × 10⁴ to 1.1 × 10⁶ cells/mL, and is more preferably 1.0 × 10⁵ to 1.1 × 10⁶ cells/mL, 1.0 × 10⁵ to 1.2 × 10⁶ cells/mL, 1.0 × 10⁵ to 1.3 × 10⁶ cells/mL, 1.0 × 10⁵ to 1.4 × 10⁶ cells/mL, 1.0 × 10⁵ to 1.5 × 10⁶ cells/mL, 1.0 × 10⁵ to 1.6 × 10⁶ cells/mL, 1.0 × 10⁵ to 1.7 × 10⁶ cells/mL, 1.0 × 10⁵ to 1.8 × 10⁶ cells/mL, 1.0 × 10⁵ to 1.9 × 10⁶ cells/mL, 1.0 × 10⁵ to 2.0 × 10⁶ cells/mL, 1.0 × 10⁵ to 2.1 × 10⁶ cells/mL, 1.0 × 10⁵ 2.2 × 10⁶ cells/mL, 1.0 × 10⁵ to 2.3 × 10⁶ cells/mL, or 1.0 × 10⁵ to 2.4 × 10⁶ cells/mL.

The bacterial content of the neutrophil activation-suppressing composition is 2.5 × 10⁶ cells/mL or more, 5.0 × 10⁶ cells/mL or more, 1.0 × 10⁷ cells/mL or more, 2.0 × 10⁷ cells/mL or more, 5.0 × 10⁷ cells/mL or more, 1.0 × 10⁸ cells/mL or more, 1.0 × 10⁹ cells/mL or more, 1.0 × 10¹⁰ cells/mL or more, or 1.0 × 10¹¹ cells/mL or more, and the upper limit is 1.0 × 10¹² cells/mL or less, 1.0 × 10¹⁵ cells/mL or less, or 1.0 × 10¹⁷ cells/mL or less.

Since the above contents are contents in an aqueous solution (cell suspension), a person skilled in the art can appropriately calculate the corresponding amounts converted into a powder, granules, an orally disintegrating tablet, a plain tablet, a coated tablet, a capsule, or a liquid formulation.

For example, when the composition of the present disclosure is in the form of a powder, granules, an orally disintegrating tablet, a plain tablet, a coated tablet, a capsule, or a liquid formulation, the content of amylolytic bacteria in the formulation can be set such that, when converted into a solution, it falls within the range of 1.0 × 10⁴ to 2.4 × 10⁶ cells/mL.

### (Target Subjects for Administration of the Composition of the Present Disclosure)

The target subjects for administration of the composition of the present disclosure are not particularly limited, but are organisms having a disease caused by insufficient neutrophil activation or a disease caused by excessive neutrophil activation, and are more preferably vertebrates having a central nervous system, and even more preferably mammals including humans and livestock, poultry, fishes, shellfish, and pets. In the case of humans, both healthy individuals and patients are included.

In addition, since a composition containing the bacterial strain has been used safely as an orally administered pharmaceutical from infants through elderly people for about 60 years, humans of all ages can be targeted.

### (Method of Administration of the Composition of the Present Disclosure)

The dosage, frequency and interval of administration in the administration method of the present disclosure are not particularly limited, and can be appropriately selected depending on conditions such as the purpose of prevention (particularly prevention of recurrence) and/or clinical treatment, the type of disease, the body weight and age of the patient, and the severity of the disease.

For example, the following dosage forms can be used, but are not particularly limited. In the case of a powder (daily dose), 75 to 150 mg as amylolytic bacterial powder is preferable, and in the case of an orally disintegrating tablet or a plain tablet (daily dose), 30 to 60 mg as amylolytic bacterial powder is preferable.

A mixture containing these bacteria, processed material thereof, or extraction residue thereof is preferably administered in the form of a powder, granules, an orally disintegrating tablet, a plain tablet, a coated tablet, a capsule, or a liquid formulation, and is administered once or twice a day or in multiple divided doses per day (morning, noon, and evening). In addition, daily administration is preferable, but administration once every several days or several times every other week is also possible.

In addition, in order to maintain the stability of the formulation, it is preferable that the amylolytic bacteria contained in the composition of the present disclosure be present in a dormant stage as spores. As a guideline for the daily intake thereof, for example, in the case of humans, it is 10³ to 10²⁰ bacterial cells (particularly spores), preferably 10⁵ to 10¹⁵ bacterial cells (particularly spores), and more preferably 10⁶ to 10¹³ bacterial cells (particularly spores). The amylolytic bacteria in the spore form germinate into the different numbers of vegetative cells as described in Example 1 depending on different environments in the living body, and then exert the function of regulating neutrophil activation.

### (Method for Regulating Neutrophil Activity)

The "method for regulating neutrophil activity" of the present disclosure comprises the step of administering the composition of the present disclosure to mammals including humans, livestock, or pets.

Specific examples of the method of administration include, in the case of a composition containing the bacteria, dispersing the bacteria in an appropriate excipient, followed by oral administration, rectal administration, enteral administration, intravaginal administration, or topical application; and, in the case of a composition containing a treated material or an extraction residue of the bacteria, diluting treated material or extraction residue to an appropriate concentration, followed by oral administration, rectal administration, enteral administration, intravaginal administration, topical application, intramuscular injection, subcutaneous injection, intradermal injection, intraperitoneal injection, sublingual administration, nasal mucosal administration, transdermal administration, inhalation, or local administration to an organ and/or tissue having a disease caused by insufficient or excessive activation of neutrophils, and the like.

### (Use of Amylolytic Bacteria for the Production of a Neutrophil Activation-Regulating Composition)

One embodiment of the present disclosure is also directed to the use of the above-described amylolytic bacteria for the production of a neutrophil activation-regulating composition containing amylolytic bacteria.

### (Use of a Neutrophil Activation-Regulating Composition Containing Amylolytic Bacteria)

One embodiment of the present disclosure is also directed to the use of above-described neutrophil activation-regulating composition containing amylolytic bacteria.

The following examples are provided to describe the present disclosure in detail, but the present disclosure is not limited to these examples.

### [EXAMPLE 1]

### (Materials and Methods)

Example 2 described below was carried out using the following materials and methods. In this Example, Bacillus subtilis TO-A was used as the amylolytic bacteria, and neutrophil-like HL-60 cells were used as a model of neutrophils.

### [Maintenance culture of HL-60 cells]

Undifferentiated HL-60 cells were maintained in Iscove's Modified Dulbecco's Medium containing 20% (v/v) FBS (IMDM medium) under conditions of 37°C, 5% CO₂, and humidity. Surface-untreated Cell Culture Flasks were used as the culture vessels.

### [Differentiation induction of neutrophil-like HL-60 cells]

After the HL-60 cell suspension concentration was adjusted to 1 × 10⁵ viable cells/mL, dimethyl sulfoxide (DMSO) was added to the IMDM medium to a final concentration of 1.25% (v/v), and the cells were cultured for 5 days under conditions of 37°C, 5% CO₂, and humidity.

### [Preparation of bacterial suspension]

Colonies of Bacillus subtilis TO-A grown on brain heart infusion agar medium were suspended in brain heart infusion liquid medium and cultured overnight with shaking under conditions of 37°C and 100 rpm. The above-cultured suspension was appropriately diluted with the same medium and further cultured with shaking. The Bacillus subtilis TO-A cultured suspension in the logarithmic growth phase was washed three times with Hanks' Balanced Salt Solution (HBSS), and then a vegetative cell suspension of Bacillus subtilis TO-A was prepared in HBSS containing 25 mM HEPES and subjected to Example 2.

### [EXAMPLE 2]

### (Confirmation of the neutrophil activation-regulating function of amylolytic bacteria)

In this Example, it was confirmed whether amylolytic bacteria could promote and/or suppress NET formation in neutrophils.

### [Induction of NETs]

The neutrophil-like HL-60 cells described above were collected using HBSS buffer containing 25 mM HEPES, the number of viable cells was counted by the trypan blue method, and a cell suspension was adjusted to 2.0 × 10⁶ viable cells/mL. Next, 50 µL of the cell suspension described above was added to each well of a 96-well plate so that the total number of neutrophil-like HL-60 cells in each well was 1.0 × 10⁵.

In addition, the logarithmic growth phase Bacillus subtilis TO-A suspension described above was measured with a particle counting analyzer CDA-1000 (Sysmex), and a suspension of Bacillus subtilis TO-A was adjusted to 2.0 × 10⁷ cells/mL using HBSS buffer containing 25 mM HEPES.

Subsequently, Bacillus subtilis TO-A was added to the wells of the 96-well plate containing neutrophil-like HL-60 cells at multiplicity of infection (MOI) of 0, 0.1, 1, 2.5, 5, or 10 bacteria per neutrophil-like HL-60 cell, by adding 0, 0.5, 5.0, 12.5, 25.0, or 50.0 µL of above Bacillus subtilis TO-A suspension, respectively. Different amounts of HBSS buffer containing 25 mM HEPES were further added to each well so that the total liquid volume in each well was adjusted to 100 µL. That is, the concentration of neutrophil-like HL-60 cells in each well was 1.0 × 10⁶ cells/mL, and the concentration of Bacillus subtilis TO-A suspension was 0, 0.1, 1, 2.5, 5, or 10 × 1.0 × 10⁶ bacterial cells/mL, respectively. After co-culturing for 1 hour under conditions of 37°C and humidity, PMA was added to a final concentration of 10 nM, and cultivation was further carried out for 3 to 4 hours.

### [Quantification of NET release]

DNase I was added to the co-cultured cell suspension in which NETs had been induced with 10 nM PMA to a final concentration of 0.4 U/mL, and the suspension was allowed to stand at room temperature for 15 minutes to partially digest the NETs. Thereafter, EGTA was added to a final concentration of 2.5 mM to stop the digestion reaction by DNase I. After centrifugation, the supernatant was used as a sample and subjected to the quantitative assay.

An anti-MPO antibody (475915-1MLCN, Merck) diluted 500-fold with carbonate-bicarbonate buffer (pH 9.5) was added to a Nunc-Immuno Plate CII (430341, Thermo Fisher Scientific) (hereinafter referred to as plate) and allowed to stand at 4°C overnight to coat the plate. The plate was washed three times with PBS containing 0.05% (v/v) Tween 20 (hereinafter referred to as Wash buffer). The solution was replaced with PBS containing 2% (w/v) BSA (hereinafter referred to as Blocking buffer), and the plate was allowed to stand at room temperature for 1 hour to further block the plate. The plate was washed three times with Wash buffer. Samples diluted to desired concentrations with Blocking buffer were added and incubated at room temperature to react for 1 hour. The plate was washed three times with Wash buffer. A peroxidase-conjugated anti-DNA antibody (clone MCA-33, 11544675001, Merck) diluted 320-fold with Blocking buffer was added and incubated at room temperature to react for 1 hour. The plate was washed three times with Wash buffer. A colorimetric reaction was carried out using a TMB Peroxidase EIA Substrate Kit (#1721066, Bio-Rad). The reaction was stopped by addition of 1N sulfuric acid solution. The absorbance at 450 nm was measured and taken as the amount of NETs released into the culture supernatant.

### [Results]

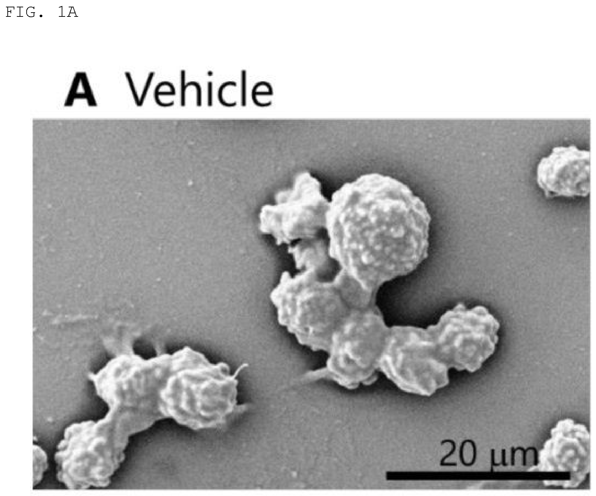

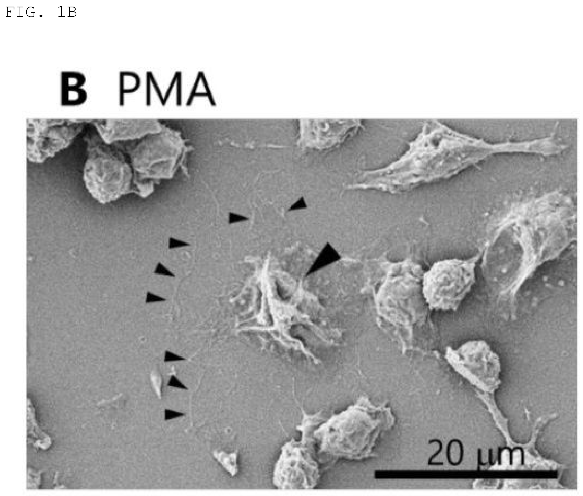

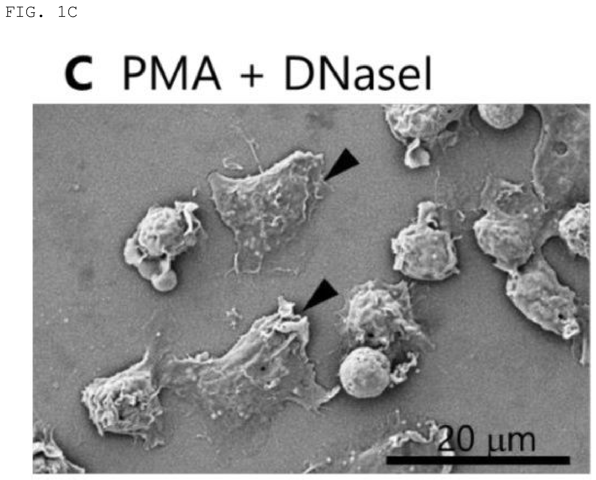

NET formation in neutrophil-like HL-60 cells was induced by PMA, specimens were prepared according to the t-butyl alcohol freeze-drying method, and observation was performed with a benchtop transmission electron microscope. In the neutrophil-like HL-60 cells to which only DMSO was added, all cells were spherical with projections on their surfaces (FIG. 1A). In contrast, in the neutrophil-like HL-60 cells to which 10 nM PMA was added, cells adhering to the bottom surface as an amorphous thin layer were observed. In addition, amorphous filamentous structures were observed around these cells (FIG. 1B). Furthermore, when 2 U/mL DNase I was applied for 15 minutes, the adherent cells were still observed, but the surrounding filamentous structures were not observed (FIG. 1C).

Thus, it was confirmed that, by adding PMA, the neutrophil-like HL-60 cells ruptured and that filamentous structures containing DNA spread around the ruptured cells.

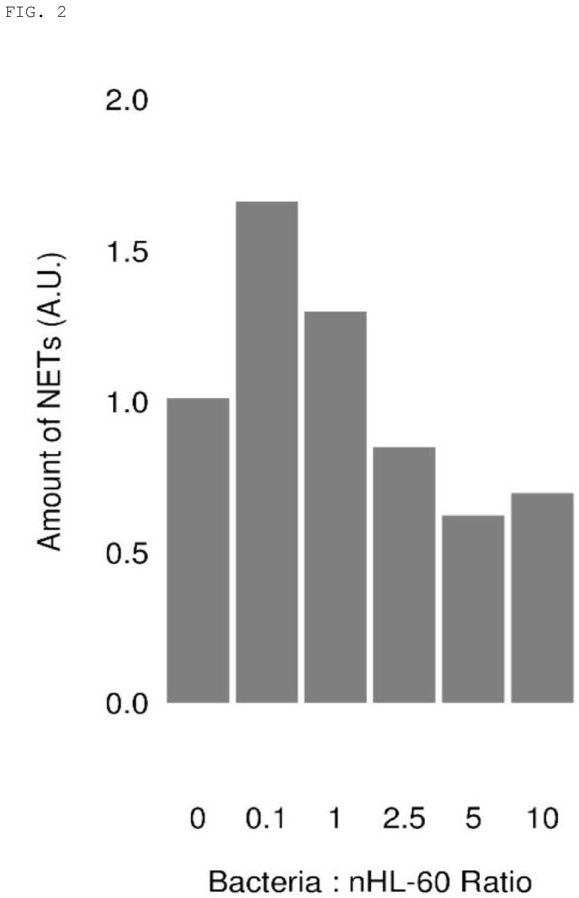

Next, Bacillus subtilis TO-A was added at a ratio (MOI) of 0, 0.1, 1, 2.5, 5, or 10 per neutrophil-like HL-60 cell, and co-culture was performed. When co-cultured at an MOI of 0.1-1 (0.1-1 × 10⁶ cells/mL of Bacillus subtilis TO-A), the amount of NETs released from the PMA-stimulated neutrophil-like HL-60 cells increased compared with that in the absence of co-cultured with Bacillus subtilis TO-A, whereas when co-cultured at MOI of 2.5-10 (2.5-10 × 10⁶ cells/mL of Bacillus subtilis TO-A), the amount of NETs decreased compared with that in the absence of co-cultured with Bacillus subtilis TO-A (FIG. 2).

From the above, it was found that when Bacillus subtilis TO-A at low-ratio (low concentration) was added and co-cultured with neutrophil-like HL-60 cells as a model of neutrophils, NET formation was promoted, whereas at a high ratio (high concentration), NET formation was suppressed.

That is, it was confirmed that amylolytic bacteria (particularly Bacillus subtilis) can regulate the neutrophil activation (promotion and/or suppression of activation) by promoting and/or suppressing NET formation in neutrophils.

### INDUSTRIAL APPLICABILITY

A neutrophil activation-regulating composition containing amylolytic bacteria can be provided.

## Claims

1. A neutrophil activation-regulating composition comprising amylolytic bacteria.

2. The composition according to claim 1, wherein the amylolytic bacteria belong to the genus Bacillus.

3. The composition according to claim 1, wherein the amylolytic bacteria are of the Bacillus subtilis species.

4. The composition according to claim 1, wherein the amylolytic bacterium is Bacillus subtilis TO-A.

5. The composition according to claim 3 or 4, wherein the neutrophil activation-regulating is promotion and/or suppression of neutrophil NET formation.

6. The composition according to claim 1, wherein the activation-regulating is promotion.

7. A therapeutic agent for a disease caused by insufficient neutrophil activation, comprising the composition according to claim 6.

8. The composition according to claim 1, wherein the activation-regulating is suppression.

9. A therapeutic agent for a disease caused by excessive neutrophil activation, comprising the composition according to claim 8.

10. A method for regulating neutrophil activity, comprising: administering the composition according to any one of claims 1 to 4 to mammals other than humans.
